Europäisches Patentamt

European Patent Office

Office européen des brevets

(19)

(11) Veröffentlichungsnummer : **0 423 484 B1**

(12) **EUROPÄISCHE PATENTSCHRIFT**

(45) Veröffentlichungstag der Patentschrift :
**03.11.93 Patentblatt 93/44**

(21) Anmeldenummer : **90117573.7**

(22) Anmeldetag : **12.09.90**

(51) Int. Cl.⁵ : **A61K 9/20, A61K 9/22**

(54) **Pressling mit retardierter Wirkstofffreisetzung.**

(30) Priorität : **16.10.89 AT 2367/89**

(43) Veröffentlichungstag der Anmeldung :
**24.04.91 Patentblatt 91/17**

(45) Bekanntmachung des Hinweises auf die
Patenterteilung :
**03.11.93 Patentblatt 93/44**

(84) Benannte Vertragsstaaten :
**CH DE GB LI**

(56) Entgegenhaltungen :
**EP-A- 0 263 490**
**AT-A- 383 270**
**FR-A- 2 070 153**

(56) Entgegenhaltungen :
**SCIENTIA PHARMACEUTICA, Band 57, 31. Dezember 1989, Seiten 317-323, Österreichische Apother-Verlages; B. KORSATKO-WABNEGG et al.: "Vergleich der Formulierungs- und Retardierungseigenschaften Biodegradabler Polyhydroxyalkanoate zur Herstellung von Matrixformulierungen"**
**PATENT ABSTRACTS OF JAPAN, Band 13, Nr. 536 (C-660)[3884], 29. November 1989; & JP-A-01 221 328 (JAPAN SYNTHETIC RUBBER CO., LTD) 04-09-1989**

(73) Patentinhaber : **PCD Polymere Gesellschaft m.b.H.**
**Danubiastrasse 21-25**
**A-2323 Schwechat-Mannswörth (AT)**

(72) Erfinder : **Korsatko-Wabnegg, Brigitta, Dr.**
**Kärntnerstrasse 432**
**A-8054 Graz (AT)**
Erfinder : **Korsatko, Werner, Dr.**
**Kärntnerstrasse 432**
**A-8054 Graz (AT)**

(74) Vertreter : **Kunz, Ekkehard, Dr.**
**Chemie Holding AG Patentwesen St. Peter-Strasse 25**
**A-4021 Linz (AT)**

EP 0 423 484 B1

## Beschreibung

Die Anmeldung betrifft einen Preßling mit retardierter Wirkstofffreisetzung zur oralen oder parenteralen Applikation von Arzneistoffen.

In US-A-3.773.919 werden pharmazeutische Depotzusammensetzungen für die parenterale Anwendung beschrieben, die einen pharmazeutischen Wirkstoff und das biologisch abbaubare Polymer Polymilchsäure (PLA), Polyglykolsäure (PGA) oder ein Copolymer aus Milchsäure und Glykolsäure enthalten und die den Wirkstoff über einen längeren Zeitraum hinweg kontrolliert abgeben. Diese Polymere weisen aber hohe elastische Eigenschaften, eine hohe elektrostatische Aufladungstendenz und schlechte Fließeigenschaften auf und sind daher nur sehr schwer zu festen Arzneiformen verpreßbar.

In EP-A-0 105 882 sind Preßlinge mit retardierter Wirkstofffreisetzung beschrieben, die aus Poly-D(-)-3-hydroxybuttersäure und einem pharmazeutischen Wirkstoff bestehen. Poly-D(-)-3-hydroxybuttersäure besitzt gute Fließeigenschaften und ist gut verpreßbar, hat aber den Nachteil, daß sie in-vivo nur sehr langsam abgebaut wird.

Es wurde nun unerwarteterveise gefunden, daß PLA durch Zusatz von Homo- oder Copolymer von D(-)-3-Hydroxybuttersäure (Poly-HB) ausgezeichnet verpreßbar wird und daß die Poly-HB in dieser Mischung in-vivo um vieles schneller abgebaut wird, als reine Poly-HB.

Gegenstand der vorliegenden Erfindung ist daher ein Preßling mit retardierter Wirkstofffreisetzung zur oralen oder parenteralen Applikation von Arzneistoffen, der dadurch gekennzeichnet ist, daß der Preßling mindestens einen festen, pharmazeutischen Wirkstoff, Polymilchsäure und ein Homo- oder Copolymer von D(-)-3-hydroxybuttersäure enthält.

Unter festen pharmazeutischen Wirkstoffen sind dabei feste, pharmazeutische Wirkstoffe oder Mischungen derselben zu verstehen, wobei die Wirkstoffe in freier Form oder in Form pharmakologisch verträglicher Salze, in Pulverform oder granuliert vorliegen können. Der erfindungsgemäße Preßling enthält etwa 5 bis 80 Gew.% Wirkstoff.

Die verwendete Polymilchsäure kann in Form des Racemates oder in Form ihrer angereicherten oder reinen Enantiomeren vorliegen. Sie besitzt üblicherweise ein Molekulargewicht von etwa 2500 bis 200000, bevorzugt ein Molekulargewicht von etwa 3000 bis 150000 und kann beispielsweise nach den in US 3.773.919 oder GB-A-932 382 geoffenbarten Verfahrensweisen hergestellt werden.

Es können sowohl Homo- als auch Copolymere von D(-)-3-Hydroxybuttersäure verwendet werden, wobei Homopolymere bevorzugt sind. Homopolymere können beispielsweise nach der in EP-A-0 144 017, Copolymere nach der in EP-A-0 052 459 beschriebenen Verfahrensweise hergestellt werden. Die üblicherweise verwendete Poly-HB besitzt ein Molekulargewicht von etwa 25000 bis 1000000, bevorzugt von etwa 50000 bis 800000.

PLA und Poly-HB liegen im erfindungsgemäßen Preßling in einem Gewichtsverhältnis von etwa 10 : 90 bis 90 : 10 vor.

Die Preßlinge können ausschließlich aus Wirkstoff, PLA und Poly-HB bestehen, es ist aber auch möglich, das sie darüber hinaus übliche galenische Hilfsstoffe, wie Gleit- und Schmierstoffe, Füllmittel oder Farbstoffe enthalten.

Die Retardwirkung des erfindungsgemäßen Preßlings ist vom Gewichtsverhältnis Wirkstoff zu Polymer, vom Gewichtsverhältnis PLA zu Poly-HB, vom Molekulargewicht der Polymeren, von der Korngröße der Bestandteile und von der Art und Weise der Herstellung des Preßlings abhängig. Dies bietet dem Fachmann die Möglichkeit, durch Auswahl und Variation dieser Parameter für jeden Wirkstoff und für jede gewünschte Applikation eine gezielte Freisetzung des Wirkstoffes über einen gewünschten Zeitraum hinweg völlig exakt einzustellen.

Die Erfindung betrifft weiterhin auch Verfahren zur Herstellung des erfindungsgemäßen Preßlings.

Zur Herstellung des Preßlings wird mindestens ein fester pharmazeutischer Wirkstoff in fester, gelöster oder suspendierter Form mit fester oder gelöster Polymilchsäure und festem oder gelöstem Homo- oder Copolymer von D(-)-3-Hydroxybuttersäure vereinigt und durchmischt, worauf das gegebenenfalls vorhandene Lösungs- oder Verdünnungsmittel abgedampft, der Rückstand gegebenenfalls getrocknet, gegebenenfalls vermahlen und verpreßt wird.

Nach einer Ausführungsform können Wirkstoff, PLA und Poly-HB jeweils in fester Form vereinigt, durchmischt und verpreßt werden.

Das Durchmischen kann bei geringen Mengen mittels Pulverreibschale und Pistill oder einer Pulvermischdose erfolgen. Große Ansätze lassen sich mit Hilfe von rotierenden Trommeln, Schaufelmischern, Tellermischern, Mischschnecken, Ribbonmischern, Konusmischern, Doppelkonusmischern und V-Mischern (Twin-Shellblender) gut durchmischen. Die erhaltenen Mischungen können direkt ohne weitere Behandlung und mit oder ohne weitere Hilfsstoffe zu Tabletten, Drageekernen oder anderen Preßlingen beliebiger Form und Größe verpreßt werden. Die Herstellung der Preßlinge ist mit allen herkömmlichen Tablettenpressen wie beispielsweise Exzenterpressen, Rundläuferpressen, hydraulischen Pressen möglich. Der Preßdtuck kann über einen Bereich von 1 bis 10 Tonnen, das sind 98,1 - 981 N pro Tablette variiert werden, wobei die Freisetzungsrate des Wirkstoffes bei einer Variation des Preßdruckes keine signifikante Abhängigkeit vom Preßdruck aufweist.

Nach einer weiteren Ausführungsform, die bevorzugt für hoch zu dosierende Wirkstoffe eingesetzt werden kann, wird der Wirkstoff in einem Wirbelschichtgranulator beispielsweise nach der in EP-A-0 285 871 beschriebenen Verfahrensweise in fester Form vorgelegt und mit einer Lösung von PLA und von Poly-HB besprüht, getrocknet und verpreßt. Der dabei erhaltene Preßling setzt den Wirkstoff im Vergleich zu einem Preßling, der durch Direktverpreßung erhalten wurde, wesentlich langsamer frei.

Nach einer anderen Ausführungsform wird der Wirkstoff in einem Verdünnungsmittel suspendiert, das gleichzeitig ein Lösungsmittel für PLA und Poly-HB ist. Solche Lösungsmittel sind beispielsweise halogenierte aliphatische Kohlenwasserstoffe, wie Methylenchlorid oder Chloroform. Die Polymeren werden in fester oder gelöster Form zugegeben und mit der Suspension des Wirkstoffes gut verrührt, wobei sich die gegebenenfalls in fester Form zugegebenen Polymeren auflösen. Das Verdünnungsmittel wird anschließend abgedampft, der Rückstand getrocknet, vermahlen und verpreßt.

Nach einer anderen Ausführungsform wird der Wirkstoff in einem Lösungsmittel, das gleichzeitig auch ein Lösungsmittel für PLA und Poly-HB ist, gelöst und mit PLA und Poly-HB in gelöster Form versetzt. Nach dem Durchmischen der Lösung wird das Lösungsmittel abgedampft und der Rückstand getrocknet, vermahlen und verpreßt.

Durch den Zusatz von Poly-HB zu PLA spielen die bekannten schlechten Verarbeitungseigenschaften von PLA unerwarteterweise keine Rolle mehr, auch wenn PLA gegenüber Poly-HB im Überschuß vorliegt. Die Mischung von PLA und Poly-HB zeigt die guten Fließeigenschaften von Poly-HB und kann somit auch ohne Zusatz irgendwelcher Hilfsstoffe problemlos zu festen Arzneiformen verpreßt werden.

Der biologische Abbau von Poly-HB geht in-vivo nur sehr langsam vor sich. Es wurde nun völlig unerwartet gefunden, daß der Abbau von Poly-HB weitaus rascher erfolgt, wenn im Preßling neben Poly-HB zusätzlich PLA vorliegt. Dazu wurden in-vivo Abbaustudien durchgeführt, in denen Preßlinge subcutan in die Nackenfalte von Mäusen implantiert, nach einem bestimmten Zeitraum entnommen und auf ihren, dem abgebauten Polymeranteil entsprechenden Gewichtsverlust überprüft wurden. Es zeigte sich, daß Poly-HB in Mischungen mit gleichen Gewichtsteilen PLA mindestens 30 mal so rasch im Körper der Versuchstiere abgebaut wird, als Poly-HB ohne Zusatz von PLA.

Beispiele:

Als Wirkstoffe wurden 2-Amino-N-(2-(2,5-dimethoxyphenyl)-2-hydroxyethyl)acetamid (Midodrin) und N'-(3-Acetyl-4-(3-((1,1-dimethylethyl)amino)-2-hydroxypoxy)phenyl)-N,N-diethylharnstoff (Celiprolol) gewählt. Das Molekulargewicht der verwendeten PLA betrug 20325, jenes der verwendeten Poly-HB 68170. Es wurde jeweils die Korngrößenfraktion von 200 bis 315 Mikrometer verwendet. Die Molekulargewichte wurden nach B. Trathnigg, C. Jorde, Journal of Chromatography, 385 (1987), Seite 17 durch Gelchromatographie bestimmt.

Zur Ermittlung der in-vitro Freisetzungsraten des Wirkstoffes wurden die erfindungsgemäßen Preßlinge den physiologischen Bedingungen entsprechend in 0,9%iger NaCl-Lösung bei 37 °C und 90 rpM in verschließbaren Braunglasflaschen in einer Infors Inkubationsschüttelmaschine RS-1-T geschüttelt und in bestimmten Zeitabständen auf die freigesetzte Wirkstoffmenge untersucht. Die quantitative analytische Erfassung der Wirkstoffe erfolgte spektralphotometrisch.

Beispiel 1

Herstellung von Retardtabletten aus Midodrin.HCl, PLA und Poly-HB durch Direktverpressung
10 mg Midodrin-HCl wurden mit 50 mg einer Mischung aus Poly-HB und PLA
A) im Verhaltnis 3 : 1
B) im Verhaltnis 1 : 1 und
C) im Verhaltnis 1 : 3
in einem Konusmischer durchmischt. Die erhaltenen Mischungen wurden bei einem Preßdtuck von 173,5 N/cm$^2$ zu Tabletten verpreßt.

Die Tabletten besaßen ein Gewicht von 60 mg, einen Durchmesser von 6 mm und eine Höhe von 2 mm. Die Bruchfestigkeit betrug von A) 7,5 kp bis C) 3,0 kp.

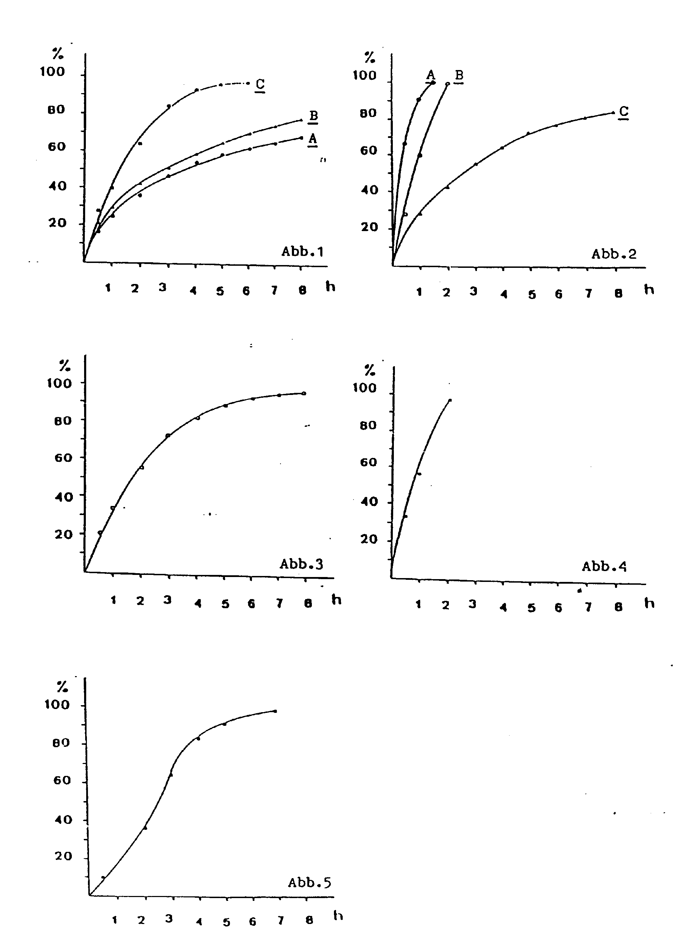

Die freigesetzte Menge an Midodrin wurde spektralphotometrisch bei einer Wellenlange von 289 nm bestimmt. Mit Hilfe der Freisetzungsuntersuchungen wurden die in Tabelle 1 zusammengestellten Werte ermittelt, die in Abb. 1 der Zeichnung graphisch dargestellt sind. Die Werte beziehen sich dabei auf die freigesetzte Menge des Wirkstoffes in Gewichtsprozent bezogen auf die ursprüngliche Gesamtmenge des Wirkstoffes in der Tablette nach der jeweiligen Zeit (in Stunden).

## Tabelle 1

| Zeit (h) | A | B | C |
|---|---|---|---|
| 0,5 | 18,27 | 20,91 | 26,80 |
| 1 | 24,58 | 28,73 | 39,23 |
| 2 | 36,08 | 41,53 | 63,50 |
| 3 | 48,30 | 50,65 | 83,51 |
| 4 | 55,20 | 58,34 | 93,13 |
| 5 | 55,80 | 64,29 | 93,41 |
| 6 | 60,93 | 69,54 | 95,03 |
| 7 | 64,92 | 73,91 | 96,77 |
| 8 | 68,53 | 77,84 | 97,25 |
| 24 | 97,34 | 99,49 | 99,84 |

Beispiel 2

Herstellung von Retardtabletten aus Celiprolol.HCl, PLA und Poly-HB durch Direktverpressung
A) 150 mg Celiprolol.HCl, 25 mg Poly-HB und 25 mg PLA (7,5 : 1,25 : 1,25)
B) 100 mg Celiprolol.HCl, 50 mg Poly-HB und 50 mg PLA (5 : 2,5 : 2,5)
C) 50 mg Celiprolol.HCl, 75 mg Poly-HB und 75 mg PLA (2,5 : 3,75 : 3,75)
wurden in einem Konusmischer durchmischt. Die erhaltenen Mischungen wurden bei einem Preßdtuck von 195,18 N/cm² zu Tabletten verpreßt. Die Tabletten besaßen ein Gewicht von 200 mg, einen Durchmesser von 8 mm und eine Hohe von 4 mm. Die Bruchfestigkeit betrug für A) 10,5 kp, für B) 9,0 kp und für C) 4,0 kp.
Die freigesetzte Menge an Celiprolol.HCl wurde spektralphotometrisch bei einer Wellenlänge von 324 nm bestimmt.

Mit Hilfe der Freisetzungsuntersuchungen wurden die in Tabelle 2 zusammengestellten Werte ermittelt, die in Abb. 2 der Zeichnung graphisch dargestellt sind. Die Werte beziehen sich dabei auf die freigesetzte Menge des Wirkstoffes in Gewichtsprozent bezogen auf die ursprüngliche Gesamtmenge des Wirkstoffes in der Tablette nach der jeweiligen Zeit (in Stunden).

## Tabelle 2

| Zeit (h) | A (%) | B (%) | C (%) |
|---|---|---|---|
| 0,5 | 66,64 | 28,24 | 18,89 |
| 1 | 90,37 | 59,82 | 28,53 |
| 1,5 | 100,00 | – | – |
| 2 | – | 99,70 | 42,60 |
| 3 | – | 100,00 | 56,43 |
| 4 | – | – | 65,12 |
| 5 | – | – | 73,33 |
| 6 | – | – | 77,52 |
| 7 | – | – | 81,71 |
| 8 | – | – | 85,95 |
| 24 | – | – | 100,00 |

Beispiel 3

Herstellung von Retardtabletten aus Celiprolol.HCl, PLA und Poly-HB durch Wirbelschichtgranulierung

Celiprolol.HCl-Granulat, erhalten durch Aufbaugranulierung mit Ethanol wurde in einem Wirbelschichtgranulator der Fa. Büchi AG, Modell Büchi 710 vorgelegt und mit 2%igen Lösungen von Poly-HB und PLA in chloroform besprüht und getrocknet. Dabei entstand ein Granulat, das 12 % Poly-HB, 12 % PLA und 76 % Celiprolol.HCl (1,2 : 1,2 : 7,6) enthielt. Das Granulat wurde anschließend bei einem Preßdruck von 195,18 N/cm² zu Tabletten verpreßt. Die Tabletten hatten ein Gewicht von 250 mg, einen Durchmesser von 8 mm und ein Höhe von 4 mm. Die Bruchfestigkeit betrug 19,5 kp, die Schütteldichte 0,45 g/ml und die Rütteldichte 0,45 g/ml. Mit Hilfe der Freisetzungsuntersuchungen wurden die in Tabelle 3 zusammengestellten Werte ermittelt, die in Abb. 3 der Zeichnung graphisch dargestellt sind. Die Werte beziehen sich dabei auf die freigesetzte Menge des Wirkstoffes in Gewichtsprozent bezogen auf die ursprüngliche Gesamtmenge des Wirkstoffes in der Tablette nach der jeweiligen Zeit (in Stunden).

## Tabelle 3

| Zeit (h) | % freigesetzter Wirkstoff |
|---|---|
| 0,5 | 21,18 |
| 1 | 33,19 |
| 2 | 56,60 |
| 3 | 73,34 |
| 4 | 83,13 |
| 5 | 90,62 |
| 6 | 94,61 |
| 7 | 97,11 |
| 8 | 98,38 |

Beispiel 4

Herstellung von Retardtabletten aus Celiprolol.HCl, PLA und Poly-HB durch Granulierung mit Hilfe des solvent-evaporation process

10 g Celiprolol.HCl wurden in 200 ml Chloroform suspendiert, mit jeweils 1 g Poly-HB und PLA versetzt und im Ultraschallbad behandelt. Anschließend wurde die Suspension - Rotovapor bei 40 °C langsam zur Trockene eingedampft. Das gebildete Granulat wurde zerkleinert und gesiebt. Die Korngrößenfraktion von 200 bis 315 nm wurde verwendet, um bei einem Preßdruck von 195,18 N/cm² Tabletten herzustellen. Die Tabletten enthielten 84 % Celiprolol.HCl, 8 % Poly-HB und 8 % PLA. Sie hatten ein Gewicht von 250 mg, einen Durchmesser von 8 mm und eine Höhe von 4,3 mm. Die Bruchfestigkeit betrug mehr als 20 kp, die Schütteldichte 0,21 g/ml und die Rütteldichte 0,31 g/ml. Mit Hilfe der Freisetzungsuntersuchungen wurden die in Tabelle 4 zusammengestellten Werte ermittelt und in Abb. 4 der Zeichnung graphisch dargestellt. Die Werte beziehen sich dabei auf die Menge des freigesetzten Wirkstoffes in Gewichtsprozent, bezogen auf die ursprüngliche Gesamtmenge des Wirkstoffes in der Tablette nach der jeweiligen Zeit (in Stunden).

## Tabelle 4

| Zeit (h) | freigesetzter Wirkstoff (%) |
|---|---|
| 0,5 | 28,04 |
| 1 | 44,31 |
| 2 | 96,85 |
| 3 | 100,00 |

Beispiel 5

Herstellung von Retardtabletten aus Celiprolol, PLA und Poly-HB durch Granulierung mit Hilfe des solvent-evaporation process

10 g Celiprolol.HCl wurden in 100 ml destilliertem Wasser gelöst und durch Zusatz von 10 ml 2 n NaOH unter Rühren in die Base übergeführt. Die wäßrige Phase wurde mehrmals mit Chloroform extrahiert, die organischen Phasen vereinigt, über Natriumsulfat getrocknet und filtriert. Die auf diese Weise hergestellte Lösung von Celiprolol in Chloroform wurde anschließend mit 1 g Poly-HB und 1 g PLA, jeweils gelöst in wenig Chloroform, unter Rühren versetzt. Das Lösungsmittel wurde am Rotovapor bei 40 °C abgedampft, wobei eine gelbliche ölige Substanz zurückblieb, die im Vakuumtrockenschrank zu einem gelblich weißen Granulat getrocknet wurde. Das Granulat wurde vorsichtig vermahlen und die Korngrößenfraktion von 200 bis 315 Mikrometer mittels einer elektrohydraulischen Presse bei einem Preßdruck von 195,18 N/cm$^2$ zu Tabletten verpreßt.

Die Tabletten enthielten 88,56 % Celiprolol, 5,72 % Poly-HB und 5,72 % PLA. Sie hatten ein Gewicht von 250 mg, einen Durchmesser von 8 mm und eine Höhe von 4,2 mm. Mit Hilfe der Freisetzungsuntersuchungen wurden die in Tabelle 5 zusammengestellten Werte ermittelt und in Abb. 5 der Zeichnung graphisch dargestellt. Die Werte beziehen sich dabei auf die freigesetzte Menge des Wirkstoffes in Gewichtsprozent bezogen auf die ursprüngliche Gesamtmenge des Wirkstoffes in der Tablette nach der jeweiligen Zeit (in Stunden).

## Tabelle 5

| Zeit (h) | freigesetzter Wirkstoff (%) |
|----------|------------------------------|
| 0,5 | 9,07 |
| 1 | 18,64 |
| 2 | 36,49 |
| 3 | 64,48 |
| 4 | 83,89 |
| 5 | 91,79 |

Beispiel 6

In-vivo Abbaustudien
A) Poly-HB
B) PLA
C) Mischungen aus A und B im Gewichtsverhältnis 1 : 1
wurden bei einem Preßdtuck von 173,5 N/cm$^2$ zu Tabletten mit einem Gewicht von 60 mg, einem Durchmesser von 6 mm und einer Höhe von 2 mm verpreßt.

Je 10 Tabletten der Zusammensetzungen A), B) und C) wurden Mäusen des Stammes NMRI, Biochemie Graz mit einem Körpergewicht von 20 bis 25 g subcutan in die Nackenfalte implantiert. Nach 5 Wochen wurden die Tabletten aus den Nackenfalten entnommen, gewaschen, bis zur Gewichtskonstanz getrocknet und die Biodegradation des Polymers quantitativ gravimetrisch und gaschromatographisch (G. Braunegg, B. Sonnleitner, R.M. Lafferty, European J.Appl. Microbiol. Biotechnol. 6, 29-37 (1978)) gemessen.

Die Tabletten der Zusammensetzung A) waren nach 5 Wochen fast unversehrt erhalten. Durchschnittlich waren nur 0,2 mg Poly-HB pro Woche im Körper abgebaut worden.

Die Tabletten der Zusammensetzungen B) und C) waren nach 5 Wochen völlig und ohne jeden Rückstand abgebaut worden. Es wurde kein Polymermaterial mehr aufgefunden.

Da die Tabletten der Zusammensetzung C) 30 mg Poly-HB enthielten, waren daher mindestens 6 mg Poly-HB pro Woche im Körper der Mäuse abgebaut worden.

**Patentansprüche**

**Patentansprüche für folgende Vertragsstaten : AT, BE, CH, DE, DK, FR, GB, IT, LI, LU, NL, SE**

1. Preßling mit retardierter Wirkstofffreisetzung zur oralen oder parenteralen Applikation von Arzneistoffen, dadurch gekennzeichnet, daß der Preßling mindestens einen festen, pharmazeutischen Wirkstoff, Polymilchsäure und ein Homo- oder Copolymer von D(-)-3-Hydroxybuttersäure enthält.

2. Preßling nach Anspruch 1, dadurch gekennzeichnet, daß die Polymilchsäure ein Molekulargewicht von 3000 bis 150000 aufweist.

3. Preßling nach Anspruch 1, dadurch gekennzeichnet, daß das Homo- oder Copolymer von D(-)-3 -Hydroxybuttersäure ein Molekulargewicht von 50000 bis 800000 aufweist.

4. Preßling nach einem der Ansprüche 1 bis 3, dadurch gekennzeichnet, daß die Polymilchsäure und das Homo- oder Copolymer von D(-)-3-Hydroxybuttersäure in einem Gewichtsverhältnis von 10 : 90 bis 90 : 10 vorliegen.

5. Preßling nach einem der Ansprüche 1 bis 4, dadurch gekennzeichnet, daß er 5 bis 80 Gewichtsprozent des festen, pharmazeutischen Wirkstoffes enthält.

6. Verfahren zur Herstellung eines Preßlings mit retardierter Wirkstofffreisetzung zur oralen oder parenteralen Applikation von Arzneistoffen, dadurch gekennzeichnet, daß mindestens ein fester pharmazeutischer Wirkstoff in fester, gelöster oder suspendierter Form mit fester oder gelöster Polymilchsäure und festem oder gelöstem Homo- oder Copolymer von D(-)-3-Hydroxybuttersäure vereinigt und durchmischt wird, worauf das gegebenenfalls vorhandene Lösungs- oder Verdünnungsmittel abgedampft, der Rückstand gegebenenfalls getrocknet, gegebenenfalls vermahlen und verpreßt wird.

7. Verfahren nach Anspruch 6, dadurch gekennzeichnet, daß mindestens ein fester pharmazeutischer Wirkstoff mit fester Polymilchsäure und einem festen Homo- oder Copolymer von D(-)-3-Hydroxybuttersäure durchmischt und verpreßt wird.

8. Verfahren nach Anspruch 6, dadurch gekennzeichnet, daß mindestens ein fester, pharmazeutischer Wirkstoff oder ein Granulat desselben in einem Wirbelschichtgranulator mit einer Lösung aus Polymilchsäure und Homo- oder Copolymer von D(-)-3-Hydroxybuttersäure besprüht, getrocknet und verpreßt wird.

9. Verfahren nach Anspruch 6, dadurch gekennzeichnet, daß mindestens ein fester, pharmazeutischer Wirkstoff in einem Lösungsmittel für Polymilchsäure und für Homo- oder Copolymer von D(-)-3-Hydroxybuttersäure suspendiert, mit gelöster Polymilchsäure und gelöstem Homo- oder Copolymer von D(-)-3-Hydroxybuttersäure durchmischt wird, worauf das Lösungsmittel abgedampft, der Rückstand getrocknet, vermahlen und verpreßt wird.

10. Verfahren nach Anspruch 6, dadurch gekennzeichnet, daß mindestens ein fester, pharmazeutischer Wirkstoff in einem Lösungsmittel für Polymilchsaure und für Homo- oder Copolymer von D(-)-3-Hydroxybuttersäure gelöst und mit gelöster Polymilchsäure und gelöstem Homo- oder Copolymer von D(-)-3-Hydroxybuttersäure versetzt und durchmischt wird, worauf das Lösungsmittel abgedampft, der Rückstand getrocknet, vermahlen und verpreßt wird.

**Patentansprüche für die folgende Vertragsstaaten : ES, GR**

1. Verfahren zur Herstellung eines Preßlings mit retardierter Wirkstofffreisetzung zur oralen oder parenteralen Applikation von Arzneistoffen, dadurch gekennzeichnet, daß mindestens ein fester pharmazeutischer Wirkstoff in fester, gelöster oder suspendierter Form mit fester oder gelöster Polymilchsäure und festem oder gelöstem Homo- oder Copolymer von D(-)-3-Hydroxybuttersäure vereinigt und durchmischt wird, worauf das gegebenenfalls vorhandene Lösungs- oder Verdünnungsmittel abgedampft, der Rückstand gegebenenfalls getrocknet, gegebenenfalls vermahlen und verpreßt wird.

2. Verfahren nach Anspruch 1, dadurch gekennzeichnet, daß eine Polymilchsäure eines Molekulargewichtes von 3000 bis 150000 eingesetzt wird.

3. Verfahren nach einem der Ansprüche 1 oder 2, dadurch gekennzeichnet, daß ein Homo- oder Copolymer von D(-)-3-Hydroxybuttersäure mit einem Molekulargewicht von 50000 bis 800000 eingesetzt wird.

4. Verfahren nach einem der Ansprüche 1 bis 3, dadurch gekennzeichnet, daß die Polymilchsäure und das Homo- oder Copolymer von D(-)-3-Hydroxybuttersäure in einem Gewichtsverhältnis von 10 : 90 bis 90 : 10 eingesetzt wird.

5. Verfahren nach einem der Ansprüche 1 bis 4, dadurch gekennzeichnet, daß 5 bis 80 Gewichtsprozent bezogen auf das Gesamtgewicht des Preßlings eines festen, pharmazeutischen Wirkstoffes eingesetzt werden.

6. Verfahren nach einem der Ansprüche 1 bis 5, dadurch gekennzeichnet, daß mindestens ein fester pharmazeutischer Wirkstoff mit fester Polymilchsäure und einem festen Homo- oder Copolymer von D(-)-3-Hydroxybuttersäure durchmischt und verpreßt wird.

7. Verfahren nach einem der Ansprüche 1 bis 5, dadurch gekennzeichnet, daß mindestens ein fester, pharmazeutischer Wirkstoff oder ein Granulat desselben in einem Wirbelschichtgranulator mit einer Lösung aus Polymilchsäure und Homo- oder Copolymer von D(-)-3-Hydroxybuttersäure besprüht, getrocknet und verpreßt wird.

8. Verfahren nach einem der Ansprüche 1 bis 5, dadurch gekennzeichnet, daß mindestens ein fester, pharmazeutischer Wirkstoff in einem Lösungsmittel für Polymilchsäure und für Homo- oder Copolymer von D(-)-3-Hydroxybuttersäure suspendiert, mit gelöster Polymilchsäure und gelöstem Homo- oder Copolymer von D(-)-3-Hydroxybuttersäure durchmischt wird, worauf das Lösungsmittel abgedampft, der Rückstand getrocknet, vermahlen und verpreßt wird.

9. Verfahren nach einem der Ansprüche 1 bis 5, dadurch gekennzeichnet, daß mindestens ein fester, pharmazeutischer Wirkstoff in einem Lösungsmittel für Polymilchsäure und für Homo- oder Copolymer von D(-)-3-Hydroxybuttersäure gelöst und mit gelöster Polymilchsäure und gelöstem Homo- oder Copolymer von D(-)-3-Hydroxybuttersäure versetzt und durchmischt wird, worauf das Lösungsmittel abgedampft, der Rückstand getrocknet, vermahlen und verpreßt wird.

## Claims

**Claims for the following Contracting States : AT, BE, CH, DE, DK, FR, GB, IT, LI, LU, NL, SE**

1. Pressing having sustained release of active compound for the oral or parenteral administration of medicaments, characterized in that the pressing contains at least one solid pharmaceutical active compound, polylactic acid and a homo- or copolymer of D(-)-3-hydroxybutyric acid.

2. Pressing according to Claim 1, characterized in that the polylactic acid has a molecular weight of 3,000 to 150,000.

3. Pressing according to Claim 1, characterized in that the homo- or copolymer of D(-)-3-hydroxybutyric acid has a molecular weight of 50,000 to 800,000.

4. Pressing according to one of Claims 1 to 3, characterized in that the polylactic acid and the homo- or copolymer of D(-)-3-hydroxybutyric acid are present in a weight ratio of 10:90 to 90:10.

5. Pressing according to one of Claims 1 to 4, characterized in that it contains 5 to 80 per cent by weight of solid pharmaceutical active compound.

6. Process for the production of a pressing having sustained release of active compound for the oral or parenteral administration of medicaments, characterized in that at least one solid pharmaceutical active compound in solid, dissolved or suspended form is combined and mixed with solid or dissolved polylactic acid and solid or dissolved homo- or copolymer of D(-)-3-hydroxybutyric acid, after which the solvent or diluent which may be present is evaporated, and the residue is optionally dried, optionally ground and compressed.

7. Process according to Claim 6, characterized in that at least one solid pharmaceutical active compound is mixed and compressed with solid polylactic acid and a solid homo- or copolymer of D(-)-3-hydroxybutyric acid.

8. Process according to Claim 6, characterized in that at least one solid pharmaceutical active compound or granules thereof are sprayed in a fluidized bed granulator with a solution of polylactic acid and homo- or copolymer of D(-)-3-hydroxybutyric acid, dried and compressed.

9. Process according to Claim 6, characterized in that at least one solid pharmaceutical active compound is suspended in a solvent for polylactic acid and for homo- or copolymer of D(-)-3-hydroxybutyric acid, mixed with dissolved polylactic acid and dissolved homo- or copolymer of D(-)-3-hydroxybutyric acid, after which the solvent is evaporated, and the residue is dried, ground and compressed.

10. Process according to Claim 6, characterized in that at least one solid pharmaceutical active compound is dissolved in a solvent for polylactic acid and for homo- or copolymer of D(-)-3-hydroxybutyric acid and dissolved polylactic acid and dissolved homo- or copolymer of D(-)-3-hydroxybutyric acid are added and mixed, after which the solvent is evaporated, and the residue is dried, ground and compressed.

**Claims for the following Contracting States : ES, GR**

1. Process for the preparation of a pressing having sustained release of active compound for the oral or parenteral administration of medicaments, characterized in that at least one solid pharmaceutical active compound in solid, dissolved or suspended form is combined and mixed with solid or dissolved polyactic acid and solid or dissolved homo- or copolymer of D-(-)-3-hydroxybutyric acid, after which the solvent or diluent which may be present is evaporated, and the residue is optionally dried, optionally ground and compressed.

2. Process according to Claim 1, characterized in that a polylactic acid is employed which has a molecular weight of 3,000 to 150,000.

3. Process according to one of Claims 1 of 2, characterized in that a homo- or copolymer of D(-)-3-hydroxybutyric acid is employed which has a molecular weight of 50,000 to 800,000.

4. Process according to one of Claims 1 to 3, characterized in that the polylactic acid and the homo- or copolymer of D(-)-3-hydroxybutyric acid are employed in a weight ratio of 10:90 to 90:10.

5. Process according to one of Claims 1 to 4, characterized in that 5 to 80 per cent by weight, based on the total weight of the pressing, of a solid pharmaceutical active compound are employed.

6. Process according to one of Claims 1 to 5, characterized in that at least one solid pharmaceutical active compound is mixed and compressed with solid polylactic acid and a solid homo- or copolymer of D(-)-3-hydroxybutyric acid.

7. Process according to one of Claims 1 to 5, characterized in that at least one solid pharmaceutical active compound or granules thereof are sprayed in a fluidized bed granulator with a solution of polylactic acid and homo- or copolymer of D(-)-3-hydroxybutyric acid, dried and compressed.

8. Process according to one of Claims 1 to 5, characterized in that at least one solid pharmaceutical active compound is suspended in a solvent for polylactic acid and for homo- or copolymer of D(-)-3-hydroxybutyric acid, mixed with dissolved polylactic acid and dissolved homo- or copolymer of D(-)-3-hydroxybutyric acid, after which the solvent is evaporated, and the residue is dried, ground and compressed.

9. Process according to one of Claims 1 to 5, characterized in that at least one solid pharmaceutical active compound is dissolved in a solvent for polylactic acid and for homo- or copolymer of D(-)-3-hydroxybutyric acid and dissolved polylactic acid and dissolved homo- or copolymer of D(-)-3-hydroxybutyric acid are added and mixed, after which the solvent is evaporated, and the residue is dried, ground and compressed.

**Revendications**

**Revendications pour les Etats contractants suivants : AT, BE, CH, DE, DK, FR, GB, IT, LI, LU, NL, SE**

1. Comprimé à libération retardée du principe actif, pour l'administration orale ou parentérale de médicaments, caractérisé en ce qu'il contient au moins un principe actif pharmaceutique solide, un poly(acide lactique) et un homo- ou copolymère d'acide D(-)-3-hydroxybutyrique.

2. Comprimé selon la revendication 1, caractérisé en ce que le poly(acide lactique) a un poids moléculaire compris entre 3 000 et 150 000.

3. Comprimé selon la revendication 1, caractérisé en ce que l'homo- ou copolymère d'acide D(-)-3-hydroxybutyrique a un poids moléculairecompris entre 50 000 et 800 000.

4. Comprimé selon une des revendications 1 à 3, caractérisé en ce que le poly(acide lactique) et l'homo- ou copolymère d'acide D(-)-3-hydroxybutyrique sont présents selon un rapport en poids compris entre 10:90 et 90:10.

5. Comprimé selon une des revendications 1 à 4, caractérisé en ce qu'il contient de 5 à 80 % en poids de principe actif pharmaceutique solide.

6. Procédé de préparation d'un comprimé à libération retardée du principe actif pour l'administration orale ou parentérale de médicaments, caractérisé en ce que l'on combine et mélange intimement au moins un principe actif pharmaceutique solide, dissous ou en suspension, avec un poly(acide lactique) solide ou dissous et un homo- ou copolymère, solide ou dissous, d'acide D(-)-3-hydroxybutyrique, après quoi on évapore le solvant ou le diluant éventuellement présent, éventuellement on fait sécher le résidu, éventuellement on le broie, et on le comprime.

7. Procédé selon la revendication 6, caractérisé en ce qu'on mélange intimement au moins un principe actif pharmaceutique solide avec un poly(acide lactique) solide et un homo- ou copolymère solide d'acide D(-)-3-hydroxybutyrique et on comprime le mélange.

8. Procédé selon la revendication 6, caractérisé en ce qu'on asperge au moins un principe actif pharmaceutique solide ou des granules de celui-ci, dans un granulateur à lit fluidisé, avec une solution de poly(acide lactique) et d'un homo- ou copolymère d'acide D(-)-3-hydroxybutyrique, on fait sécher le mélange et on le comprime.

9. Procédé selon la revendication 6, caractérisé en ce qu'on met au moins un principe actif pharmaceutique solide en suspension dans un solvant pour le poly(acide lactique) et pour l'homo- ou copolymère d'acide D(-)-3-hydroxybutyrique, on mélange intimement la suspension avec un poly(acide lactique) dissous et un homo- ou copolymère dissous d'acide D(-)-3-hydroxybutyrique, ensuite on évapore le solvant, on fait sécher le résidu, on le broie et on le comprime.

10. Procédé selon la revendication 6, caractérisé en ce qu'on dissout au moins un principe actif pharmaceutique solide dans un solvant pour le poly(acide lactique) et pour l'homo- ou copolymère d'acide D(-)-3-hydroxybutyrique, on ajoute le poly(acide lactique) dissous et l'homo- ou copolymère dissous d'acide D(-)-3-hydroxybutyrique et on les mélange intimement, ensuite on évapore le solvant, on fait sécher le résidu, on le broie et on le comprime.

**Revendications pour les Etats contractants suivants : ES, GR**

1. Procédé de préparation d'un comprimé à libération retardée du principe actif pour l'administration orale ou parentérale de médicaments, caractérisé en ce que l'on combine et mélange intimement au moins un principe actif pharmaceutique solide, dissous ou en suspension, avec un poly(acide lactique) solide ou dissous et un homo- ou copolymère, solide ou dissous, d'acide D(-)-3-hydroxybutyrique, après quoi on évapore le solvant ou le diluant éventuellement présent, éventuellement on fait sécher le résidu, éventuellement on le broie, et on le comprime.

**2.** Procédé selon la revendication 1, caractérisé en ce qu'on utilise un poly(acide lactique) d'un poids moléculaire compris entre 3 000 et 150 000.

**3.** Procédé selon la revendication 1 ou 2, caractérisé en ce qu'on utilise un homo- ou copolymère d'acide D(-)-3-hydroxybutyrique d'un poids moléculaire compris entre 50 000 et 800 000.

**4.** Procédé selon une des revendications 1 à 3, caractérisé en ce qu'on utilise le poly(acide lactique) et l'homo- ou copolymère d'acide D(-)-3-hydroxybutyrique selon un rapport en poids compris entre 10:90 et 90:10.

**5.** Procédé selon une des revendications 1 a 4, caractérisé en ce qu'on utilise de 5 à 80 % en poids de principe actif pharmaceutique solide, par rapport au poids total du comprimé.

**6.** Procédé selon une des revendications 1 à 5, caractérisé en ce qu'on mélange intimement au moins un principe actif pharmaceutique solide avec un poly(acide lactique) solide et un homo- ou copolymère solide d'acide D(-)-3-hydroxybutyrique et on comprime le mélange.

**7.** Procédé selon une des revendications 1 à 5, caractérisé en ce qu'on asperge au moins un principe actif pharmaceutique solide ou des granules de celui-ci, dans un granulateur à lit fluidisé, avec une solution de poly(acide lactique) et d'un homo- ou copolymère d'acide D(-)-3-hydroxybutyrique, on fait sécher le mélange et on le comprime.

**8.** Procédé selon une des revendications 1 à 5, caractérisé en ce qu'on met au moins un principe actif pharmaceutique solide en suspension dans un solvant pour le poly(acide lactique) et pour l'homo- ou copolymère d'acide D(-)-3-hydroxybutyrique, on mélange intimement la suspension avec un poly(acide lactique) dissous et un homo- ou copolymère dissous d'acide D(-)-3-hydroxybutyrique, ensuite on évapore le solvant, on fait sécher le résidu, on le broie et on le comprime.

**9.** Procédé selon une des revendications 1 à 5, caractérisé en ce qu'on dissout au moins un principe actif pharmaceutique solide dans un solvant pour le poly(acide lactique) et pour l'homo- ou copolymère d'acide D(-)-3-hydroxybutyrique, on ajoute le poly(acide lactique) dissous et l'homo- ou copolymère dissous d'acide D(-)-3-hydroxybutyrique et on les mélange intimement, ensuite on évapore le solvant, on fait sécher le résidu, on le broie et on le comprime.

Abb.1

Abb.2

Abb.3

Abb.4

Abb.5